# EUROPÄISCHE PATENTSCHRIFT

(11) **EP 3 628 229 B1**
(45) Veröffentlichungstag und Bekanntmachung des Hinweises auf die Patenterteilung: **21.07.2021**
(21) Anmeldenummer: 18197212.6
(22) Anmeldetag: 27.09.2018
(51) Int. Cl.: A61B 6/02, A61B 6/00

(54) **ERZEUGUNG EINES ERGEBNISBILDES**
GENERATION OF A FINAL IMAGE
GÉNÉRATION D'UNE IMAGE DE RÉSULTAT

(43) Veröffentlichungstag der Anmeldung: 01.04.2020
(73) Patentinhaber: Siemens Healthcare GmbH, 91052 Erlangen (DE)
(72) Erfinder: Radicke, Marcus, 90587 Veitsbronn (DE)

(56) Entgegenhaltungen:
- DE-A1-102010 011 662
- DE-A1-102010 011 663
- US-A1- 2011 129 067
- US-A1- 2016 374 187

## Beschreibung

Die Erfindung betrifft ein Verfahren zur Erzeugung eines Ergebnisbildes mittels eines Tomosynthesegeräts, eine Ausgleichsvorrichtung, ein Tomosynthesegerät und ein Tomosynthesesystem.

Medizinische Untersuchungen einer Brust, insbesondere die einer menschlichen weiblichen Brust, werden in der Regel durchgeführt, um bösartige Veränderungen des Brustgewebes gezielt und sicher feststellen bzw. diagnostizieren zu können. Eine gängige Methode ist die Mammographie, um solch eine Untersuchung durchführen zu können.

Ein herkömmliches Mammographiegerät besteht aus einer an einem Stativ angeordneten Röntgenquelle, einer Detektor- und einer Kompressionseinheit. Ein U-förmiger Träger, an dessen Enden einerseits die Röntgenquelle sowie andererseits die Detektoreinheit angeordnet sind, ist an dem Stativ drehbar befestigt. Vor einer Untersuchung wird die Brust üblicherweise mittels eines Kompressionselementes komprimiert. Bei einer cranio-kaudalen Projektion oder bei einer mediolateraloblique-Projektion wird der Träger einmal vertikal ausgerichtet und bei der zweiten Aufnahme um beispielsweise 45 Grad geneigt. Beim Wechsel der Projektionen wird der U-förmige Träger um seine zentrale Achse geschwenkt.

Neben der herkömmlichen Mammographie gewinnt die Tomosynthese zunehmend an Bedeutung. Bei der Tomosynthese wird der Detektor mit der Kompressionseinheit von dem zentralen U-förmigen Träger entkoppelt. Bei diesem Untersuchungsverfahren wird die komprimierte Brust ortsfest gehalten und mit einer Röntgenquelle die zum Beispiel entlang eines Kreisbogensegmentes verfahren wird aus unterschiedlichen Richtungen durchleuchtet. Die einzelnen so akquirierten Projektionsaufnahmen werden in einer Recheneinheit zwischengespeichert und anschließend zu einem Volumenbild rekonstruiert.

Die 2D-Röntgenaufnahmen der Mammographie ermöglichen eine gute Diagnose. Die Befundbarkeit des Mammographiebildes stößt jedoch an ihre Grenzen, wenn unterschiedliche Gewebeschichten in Richtung des Röntgenstrahls überlagert werden oder das Gewebe sehr dicht ist. Eine Aussage über die Lage von Details entlang der Strahlungsrichtung ist in der Regel nur eingeschränkt möglich. Um diese Einschränkungen zu minimieren, wird auf die 3D-Bildgebung zurückgegriffen.

Die Tomosynthese nutzt im Gegensatz zu einem 3D-Computertomogramm einen eingeschränkten Winkelbereich bei der Akquisition. Bei der Tomosynthese wird die Röntgenquelle z.B. entlang eines Kreisbogenabschnittes um die Brust bewegt. Durch die vorgegebene Hauptstrahlrichtung der auf einer vorgegebenen Bahn sich bewegenden Röntgenquelle können Strukturen entlang der Hauptstrahlrichtung verschmiert werden.

Aus der Praxis sind inzwischen Tomosynthesesysteme mit einer Mehrzahl von Röntgenquellen bekannt, welche matrixförmig stationär in Zeilen und Spalten innerhalb eines Feldes angeordnet sind. Eine Ansteuerungseinheit steuert die Röntgenquellen des Feldes derart, dass die Röntgenquellen nacheinander in Abhängigkeit von ihrer Zeile und Spalte im Feld Röntgenstrahlen abgeben. Folgend wird aus den einzelnen so akquirierten Projektionsaufnahmen im Rahmen Rekonstruktionsverfahrens ein Gesamtvolumenbild errechnet. Ein solches Gerät ist z.B. aus der DE 10 2010 011 662 A1 bekannt.

Bei dem zuvor beschriebenen Verfahren ist jedoch nachteilig, dass es bereits nicht mehr angewendet werden kann, sobald eine der Röntgenquellen nicht mehr voll funktionstüchtig ist. Es ist daher eine Aufgabe der vorliegenden Erfindung, die Ausfallzeiten beim Betrieb eines Tomosynthesegeräts mit mehreren stationären Röntgenquellen zu reduzieren.

Diese Aufgabe wird durch ein Verfahren zur Erzeugung eines Ergebnisbildes mittels eines Tomosynthesegeräts gemäß Patentanspruch 1, eine Ausgleichsvorrichtung gemäß Patentanspruch 8, ein Tomosynthesegerät gemäß Patentanspruch 9 und ein Tomosynthesesystem gemäß Patentanspruch 10 gelöst.

Das eingangs genannte Verfahren dient dazu, ein Ergebnisbild mittels eines Tomosynthesegeräts zu erzeugen, welches eine Mehrzahl von stationären Röntgenquellen aufweist. Das Verfahren umfasst dabei zumindest folgende Schritte: Zumindest eine mangelhafte Röntgenquelle wird detektiert, eine Rekonstruktion wird zum Ausgleich der mangelhaften Röntgenquelle angepasst und, das Ergebnisbild wird mittels der angepassten Rekonstruktion erzeugt.

Bei dem Ergebnisbild handelt es sich um ein beliebiges, mittels eines Tomosynthesegeräts erzeugbares Bild. Es kann zweidimensional oder dreidimensional sein. Das Ergebnisbild wird dabei üblicherweise aus einer Anzahl von Projektionsaufnahmen rekonstruiert. Dabei wird z. B. jeweils eine der Projektionsaufnahmen aus jeweils einer Richtung wie eingangs beschrieben unter der Verwendung jeweils einer der stationären, vorzugsweise in einer Matrix angeordneten, Röntgenquellen akquiriert. Im Rahmen einer Dual- bzw. Multi-Energy-Bildgebung können aber auch mehrere Projektionsaufnahmen mit unterschiedlichen Energiespektren der Röntgenstrahlung aus je einer Richtung aktiviert werden. Das Ergebnisbild kann beispielsweise in Form eines synthetischen Mammogramms und/oder als Volumenbild, z. B. in Form eines Schichtbild-Datenstapels, mittels aus der Praxis bekannten Verfahren rekonstruiert werden.

Die Rekonstruktion beschreibt dabei einen Rekonstruktionsprozess bzw. ein Rekonstruktionsverfahren. Die Rekonstruktion bezeichnet also einen Teilvorgang bei der Erzeugung des Ergebnisbildes, in dem die im Vorhinein aus unterschiedlichen Winkeln akquirierten Projektionsaufnahmen in Verbindung mit ihrer Winkelinformation als Eingangsdaten verwendet werden, um das Ergebnisbild zu ermitteln.

Bei einer regulären Rekonstruktion liegt ein vollständiger bzw. normaler Satz von Projektionsaufnahmen vor, die aus allen für eine definierte Untersuchung vorgesehenen Projektionsrichtungen bzw. unter Verwendung aller entsprechend benötigten Röntgenquellen akquiriert wurden.

Ist jedoch - wie im erfindungsgemäßen Fall - eine der Röntgenquellen mangelhaft, kann aus ihrer Richtung keine gleichwertige bzw. keine geeignete oder gar keine Projektionsaufnahme akquiriert und als Eingangsdaten für die Rekonstruktion verwendet werden. Daher liegt erfindungsgemäß nur ein abweichender Satz von Projektionsaufnahmen vor, der als Basis für die Rekonstruktion dient.

Die Erfindung betrifft also kein normales bzw. reguläres Tomosyntheseverfahren unter Verwendung einer Mehrzahl von ortsfesten Röntgenquellen, wie beim Stand der Technik, sondern ein davon aufgrund zumindest einer mangelhaften, nicht voll funktionstüchtigen Röntgenquelle abweichendes Verfahren.

Die zumindest eine mangelhafte Röntgenquelle wird detektiert bzw. ermittelt, d.h. eine Funktionsabweichung bzw. ein Defekt der Röntgenquellen wird festgestellt. Dies kann beispielsweise über die Messung eines Leistungsabfalls bzw. eine Widerstandsmessung an der Röntgenquellen erfolgen. Alternativ oder zusätzlich können die Röntgenquellen im Rahmen einer Kalibrierungsmessung verglichen und auf diese Weise Abweichungen einzelner Röntgenquellen festgestellt werden.

Dass "zumindest eine" mangelhafte Röntgenquelle detektiert wird, bedeutet, dass es sich auch um eine beliebige (kleine) Anzahl, d.h. beispielsweise auch zwei, drei, vier, fünf oder mehr, mangelhafter Röntgenquellen handeln kann. Dabei ist je nach Anwendung abzuwägen, bis zu wie viel mangelhaften Röntgenquellen die Qualität des erzeugten Ergebnisbildes für eine Befundung noch ausreichend ist.

Als Anpassung der Rekonstruktion wird dabei eine Änderung gegenüber der normalen Rekonstruktion verstanden, deren Eingangsdaten, wie regulär für die Untersuchung vorgesehen mittels eines voll funktionstüchtigen Tomosynthesegeräts akquiriert wurden. Die Anpassung erfolgt dabei insbesondere in Abhängigkeit von der detektierten mangelhaften Röntgenquelle, um deren Wirkung für die Bildgebung auszugleichen. D. h. der negative Effekt, den die mangelhafte Röntgenquelle auf die Bildgebung haben würde, wird erfindungsgemäß möglichst stark durch die Anpassung der Rekonstruktion verringert. Im Rahmen der Anpassung der Rekonstruktion können z. B. grundsätzliche Elemente des Verfahrens, wie beispielsweise eine für die Tomosynthese geeignete gefilterte Rückprojektion (FPB, filtered backprojection) oder dergleichen gewählt werden. Ferner können nach Bedarf gegebenenfalls Pre- oder Post-Processing-Schritte durchgeführt werden. Es kann aber auch eine Vielzahl von Parametern, wie beispielsweise die Gewichtung einzelner Projektionsaufnahmen, angepasst werden.

Die Erzeugung des Ergebnisbildes kann zeitlich und/oder räumlich beanstandet zur Anpassung der Rekonstruktion erfolgen. D.h. im Rahmen der Anpassung der Rekonstruktion können Prozesse, Einstellungen und/oder Parameterwerte ermittelt werden, die später bzw. zeitlich versetzt und/oder andernorts bzw. in einer räumlich getrennten Rekonstruktionseinheit im Rahmen der Rekonstruktion verwendet werden. Die Erzeugung des Ergebnisbildes kann aber auch im direkten Anschluss an die vorherigen Verfahrensschritte und/oder in einer Rechnereinheit erfolgen, in der Anpassungseinheit und Rekonstruktionseinheit und/oder Steuerungseinrichtung integriert sind.

Die eingangs genannte Ausgleichsvorrichtung dient zum Ausgleich einer mangelhaften Röntgenquelle bei einem Tomosynthesegerät, das eine Mehrzahl von stationären Röntgenquellen aufweist. Sie umfasst eine Steuerungseinrichtung mit einer Detektionseinheit, die ausgebildet ist, eine mangelhafte Röntgenquelle zu detektieren. Die Ausgleichsvorrichtung umfasst ferner eine Anpassungseinheit, die ausgebildet ist, eine Rekonstruktion zum Ausgleich der mangelhaften Röntgenquelle anzupassen, und eine Rekonstruktionseinheit, die ausgebildet ist, ein Ergebnisbild mittels der angepassten Rekonstruktion zu erzeugen.

Die Steuerungseinrichtung, die Anpassungseinheit und die Rekonstruktionseinheit wirken also im Rahmen der Erfindung zusammen und umfassen gemeinsam als Ausgleichsvorrichtung alle Komponenten zur Durchführung eines erfindungsgemäßen Verfahrens zur Erzeugung eines Ergebnisbildes.

Das eingangs genannte Tomosynthesegerät weist eine Mehrzahl von stationären Röntgenquellen und eine erfindungsgemäße Ausgleichsvorrichtung auf, welche zur Akquisition von Projektionsaufnahmen dienen. Dabei heißt Mehrzahl, zumindest mehr als eine Röntgenquelle, jedoch mindestens auch mehr Röntgenquellen als für eine den Anforderungen an die Befundung entsprechende Bildgebung notwendig sind. Das Tomosynthesegerät weist also z. B. 25 Röhren Röntgenquellen auf. Bei geringeren Anforderungen wäre eine Bildgebung beispielsweise auch mit zumindest der Hälfte der ursprünglich vorgesehenen Röntgenquellen möglich.

Die Röntgenquellen sind stationär bzw. ortsfest. D.h. sie sind zumindest während einer Untersuchung in Relation zu einem Untersuchungsobjekt, wie beispielsweise einem Patienten, unbewegt und sind insbesondere in einem Array bzw. in einer Matrix unbeweglich zueinander angeordnet. Als Röntgenquellen können grundsätzlich alle geeigneten, d. h. ausreichend kleinen Röntgenquellen verwendet werden. Sie können beispielsweise als Feldemitterröhren, insbesondere in Form von Carbo-Nano-Tubes ausgebildet sein.

Das eingangs genannte Tomosynthesesystem umfasst ein erfindungsgemäßes Tomosynthesegerät sowie eine erfindungsgemäße

Rekonstruktionseinrichtung. Daneben kann es noch weitere Komponenten wie beispielsweise eine Schnittstelle zu einem Netzwerk aufweisen. Darüber können z. B. Daten, die die mangelhafte Röntgenquelle betreffen übermittelt werden. Dadurch kann ggf. automatisch Servicepersonal informiert werden, das eine Reparatur oder einen Austausch der defekten Röntgenquelle vornehmen kann. Die Daten können aber beispielsweise auch genutzt werden, um auf dem nicht mehr vollständig funktionsfähigen Tomosynthesegerät bis zur Reparatur bzw. Instandsetzung nur noch Untersuchungen mit geringeren Anforderungen an die Qualität der Bildgebung durchzuführen und Untersuchungen mit höheren Anforderungen umzuverteilen.

Die erfindungsgemäße Ausgleichsvorrichtung kann vorteilhafterweise in bereits existenten Tomosynthesegeräten nachgerüstet werden. Ebenso ist es jedoch möglich neu zu fertigende Tomosynthesegeräte bereits bei der Fertigung mit einer erfindungsgemäßen Ausgleichsvorrichtung auszustatten.

Die wesentlichen Komponenten der erfindungsgemäßen Ausgleichsvorrichtung können zum überwiegenden Teil in Form von Softwarekomponenten ausgebildet sein. Grundsätzlich können diese Komponenten aber auch zum Teil, insbesondere wenn es um besonders schnelle Berechnungen geht, in Form von softwareunterstützter Hardware, beispielsweise FPGAs oder dergleichen, realisiert sein. Ebenso können die benötigten Schnittstellen, beispielsweise wenn es nur um eine Übernahme von Daten aus anderen Softwarekomponenten geht, als Softwareschnittstellen ausgebildet sein. Sie können aber auch als hardwaremäßig aufgebaute Schnittstellen ausgebildet sein, die durch geeignete Software angesteuert werden.

Insbesondere kann die erfindungsgemäße Ausgleichsvorrichtung Teil eines Benutzerterminals eines Tomosynthesegeräts sein.

Eine weitgehend softwaremäßige Realisierung hat den Vorteil, dass auch schon bisher verwendete Ausgleichsvorrichtungen auf einfache Weise durch ein Software-Update nachgerüstet werden können, um auf die erfindungsgemäße Weise zu arbeiten. Insofern wird die Aufgabe auch durch ein entsprechendes Computerprogrammprodukt mit einem Computerprogramm gelöst, welches direkt in eine Speichereinrichtung einer Ausgleichsvorrichtung eines Tomosynthesegeräts ladbar ist, mit Programmabschnitten, um alle Schritte des erfindungsgemäßen Verfahrens auszuführen, wenn das Programm in der Ausgleichsvorrichtung ausgeführt wird. Ein solches Computerprogrammprodukt kann neben dem Computerprogramm gegebenenfalls zusätzliche Bestandteile wie z. B. eine Dokumentation und/oder zusätzliche Komponenten, auch Hardware-Komponenten, wie z.B. Hardware-Schlüssel (Dongles etc.) zur Nutzung der Software, umfassen.

Zum Transport zur Ausgleichsvorrichtung und/oder zur Speicherung an oder in der Ausgleichsvorrichtung kann ein computerlesbares Medium, beispielsweise ein Memorystick, eine Festplatte oder ein sonstiger transportabler oder fest eingebauter Datenträger dienen, auf welchem die von einer Rechnereinheit der Ausgleichsvorrichtung einlesbaren und ausführbaren Programmabschnitte des Computerprogramms gespeichert sind. Die Rechnereinheit kann z.B. hierzu einen oder mehrere zusammenarbeitende Mikroprozessoren oder dergleichen aufweisen.

Weitere, besonders vorteilhafte Ausgestaltungen und Weiterbildungen der Erfindung ergeben sich aus den abhängigen Ansprüchen sowie der nachfolgenden Beschreibung, wobei die unabhängigen Ansprüche einer Anspruchskategorie auch analog zu den abhängigen Ansprüchen oder Beschreibungsteilen einer anderen Anspruchskategorie weitergebildet sein können und insbesondere auch einzelne Merkmale verschiedener Ausführungsbeispiele bzw. Varianten zu neuen Ausführungsbeispielen bzw. Varianten kombiniert werden können.

Bevorzugt werden bei der Anpassung der Rekonstruktion die Gewichtungen der Röntgenquellen bzw. die Gewichtungen der von den einzelnen Röntgenquellen akquirierten Projektionsaufnahmen umverteilt. D. h. zum Ausgleich der nicht oder nur noch teilweise funktionierenden Röntgenquelle, deren Projektionsaufnahmen nicht mehr oder nur noch zum Teil bei der Ermittlung des Ergebnisbildes verwendet wird, werden die voll funktionstüchtigen Röntgenquellen stärker gewichtet.

Besonders bevorzugt werden die funktionstüchtigen Röntgenquellen stärker gewichtet, die benachbart zu der mangelhaften Röntgenquellen angeordnet sind. Ganz besonders bevorzugt werden die zur mangelhaften Röntgenquellen direkt benachbarten funktionstüchtigen Röntgenquellen noch stärker gewichtet als die übrigen funktionstüchtigen Röntgenquellen.

Weiterhin bevorzugt können auch indirekt benachbarte Röntgenquellen, zwischen denen und der mangelhaften Röntgenquelle also z. B. eine, zwei oder mehrere funktionstüchtige angeordnet sind, entsprechend stärker gewichtet werden.

Durch die stärkere Gewichtung benachbarter Röntgenquellen, kann ein Winkelbereich, der aufgrund der mangelhaften Röntgenquelle schwächer in der Rekonstruktion repräsentiert ist, vorteilhafterweise ausgeglichen werden.

Als Röntgenstrom wird ein Elektronenstrom bezeichnet, der zur Erzeugung von Röntgenstrahlung von einer Kathode der Röntgenquellen zu einer Anode der Röntgenquellen beschleunigt wird und durch Wechselwirkung mit dem Material der Anode Röntgenstrahlung mit einem Röntgenspektrum erzeugt. Der Röntgenstrom und eine Röhrenspannung, die zwischen der Kathode und der Anode angelegt wird, definieren allgemein die Intensität einer Röntgenabbildung und somit die Intensität der Projektionsaufnahmen. Daher werden die Röntgenströme der funktionsfähigen Röntgenquellen bevorzugt erhöht, um vorteilhafterweise Projektionsaufnahmen und somit auch ein Ergebnisbild mit gleicher Intensität wie bei einer regulären Akquisition zu erhalten. Dass die Röntgenströme erhöht werden, bedeutet dabei, dass ihre Parameterwerte für eine - nicht von dem Verfahren umfasste - Bildakquisition höher eingestellt werden. Besonders bevorzugt werden die Röntgenströme in Abhängigkeit von der Anzahl der mangelhaften Röntgenquellen erhöht. D.h. je mehr Röntgenquellen mangelhaft bzw. ausgefallen sind, umso stärker werden die Rentenströme der funktionstüchtigen Röntgenquellen erhöht.

Ganz besonders bevorzugt werden die Röntgenströme der Röntgenquellen, die benachbart zu der mangelhaften Röntgenquelle angeordnet sind, stärker erhöht. Wie oben bereits erläutert, können auch die Röntgenströme von indirekten Nachbarn der mangelhaften Röntgenquellen erhöht werden.

Wie oben bereits erwähnt, wird die Intensität der Röntgenabbildung unter anderem auch durch die Röhrenspannung definiert. Um die Intensität der Bildung auch bei einer mangelhaften Röntgenquelle gleich zu halten, werden daher bevorzugt die Röhrenspannungen der funktionsfähigen Röntgenquellen erhöht. Dabei ist jedoch abzuwägen, inwieweit mit der Erhöhung der Röhrenspannung auch eine Verschiebung des Röntgenspektrums einhergeht, die unerwünschten Einfluss auf die Bildgebung haben kann. Die Röhrenspannungen werden daher insbesondere dann erhöht, wenn eine Erhöhung der Röntgenstrahlung aus anderen Gründen nicht möglich ist. Auch hier bedeutet die Erhöhung der Röhrenspannung, dass ihre Parameterwerte für eine - nicht von dem Verfahren umfasste - Bildakquisition höher eingestellt werden.

Wie oben bereits anhand der Gewichtung der Röntgenquellen bzw. der Erhöhung des Röntgenstroms beschrieben, wird auch die Röntgenspannung besonders bevorzugt bei benachbarten Röntgenquellen, also insbesondere direkt und/oder indirekt benachbarte Röntgenquellen, erhöht.

Vorzugsweise wird eine Gesamtdosis gleich gehalten, wie bei einer regulären Untersuchung mittels eines voll funktionsfähigen Tomosynthesegeräts. D.h., dass die Röntgenintensität mittels der oben beschriebenen jeweiligen Erhöhung des Röntgenstroms und/oder der Röhrenspannung der einzelnen funktionstüchtigen Röntgenquellen bevorzugt in etwa so eingestellt wird, dass sie der Dosis entspricht, die bei einer regulären Untersuchung auf ein Untersuchungsobjekt einwirkt.

Bevorzugt wird eine Fehlerinformation betreffend die detektierte mangelhafte Röntgenquelle erzeugt. Die Fehlerinformation bzw. die Fehlermeldung wird besonders bevorzugt über ein Netzwerk übermittelt. Wie oben bereits erläutert, kann dadurch Service-Personal informiert werden und/oder eine Umverteilung von Untersuchungen mit höheren Anforderungen an die Bildgebung auf andere Modalitäten erfolgen.

Die Erfindung wird im Folgenden unter Hinweis auf die beigefügten Figuren anhand von Ausführungsbeispielen noch einmal näher erläutert. Dabei sind in den verschiedenen Figuren gleiche Komponenten mit identischen Bezugsziffern versehen. Die Figuren sind in der Regel nicht maßstäblich. Es zeigen:
FIG 1 eine grob schematische Darstellung eines Ausführungsbeispiels eines erfindungsgemäßen Tomosynthesesystems und
FIG 2 ein Blockschaltbild eines Ausführungsbeispiels eines erfindungsgemäßen Verfahrens zur Erzeugung eines Ergebnisbilds.

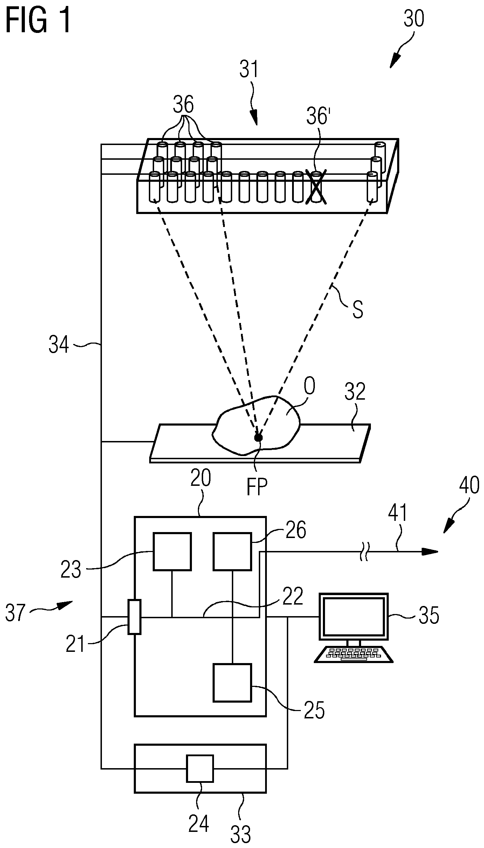

FIG 1 zeigt beispielhaft und grob schematisch ein erfindungsgemäßes Tomosynthesesystem 40, welches ein Ausführungsbeispiel eines erfindungsgemäßen Tomosynthesegeräts 30 umfasst. Das Tomosynthesegerät 30 weist eine matrixförmige Röntgenquellenanordnung 31 mit einem Feld von Röntgenquellen 36, 36' auf. In der Röntgenquellenanordnung 31 sind beispielsweise drei Zeilen und eine Mehrzahl von Spalten mit einer Mehrzahl von Röntgenquellen 36, 36' angeordnet. Die Röntgenquellenanordnung 31 kann auch einwärts gewölbt sein.

Das Tomosynthesegerät 30 weist zudem ein Detektor 32 auf, mit dem die Röntgenquellenanordnung 31 stationär mit einem hier nicht explizit dargestellten Stativ verbunden sein kann.

Zwischen der Röntgenquellenanordnung 31 und dem Detektor 32 ist für eine Untersuchung ein Untersuchungsobjekt O, wie beispielsweise der Brust einer Patientin, angeordnet. Die stationären Röntgenquellen 36, 36' sind dabei auf einen Fokuspunkt FP in dem Untersuchungsobjekt O ausgerichtet.

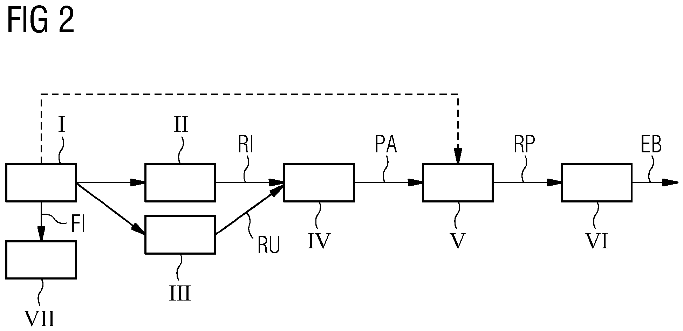

Im Betrieb senden die Röntgenquellen 36 in einem von dem erfindungsgemäßen Verfahren nicht umfassten Akquisitionsschritt nacheinander jeweils Röntgenstrahlung S aus. Die Röntgenstrahlung S durchdringt das Untersuchungsobjekt O und wird von dem Detektor 32 für jede Röntgenquelle 36 einzeln als Projektionsaufnahme PA (siehe FIG 2) erfasst.

Die Anordnung bringt den Vorteil mit sich, dass keinerlei mechanische Bewegung von Röntgenröhren 36, 36' und/oder Detektor 32 nötig wird und daher keine Fokusverwischung auftritt welche die Auflösung der Bilder verringert.

Das Tomosynthesegerät 30 umfasst ferner eine Steuerungseinrichtung 20 sowie eine Rekonstruktionseinheit 33. Die Röntgenquellenanordnung 31, der Detektor 32, die Steuerungseinrichtung sowie die Rekonstruktionseinheit 33 sind beispielsweise über einen gemeinsamen Bus 34, zum Austausch von Daten verbunden ist. Die ausgetauschten Daten können dabei z. B. Steuerungsdaten, Kontrolldaten und/oder die Daten der vom Detektor 32 erfassten Projektionsaufnahmen PA umfassen.

Die Steuerungseinrichtung 20 weist neben weiteren für eine Steuerung eines Tomosynthesegeräts üblichen (hier nicht dargestellten) Komponenten unter anderem eine Detektionseinheit 23, eine Röhrenspannungsregelungseinheit 25 sowie eine Röntgenstromregelungseinheit 26 sowie eine Schnittstelle 21 auf. Die einzelnen Komponenten der Steuerungseinrichtung 20 sind dabei untereinander über einen internen Bus 22 miteinander zum Austausch von Daten verbunden; eine Verbindung zu den übrigen Komponenten des Tomosynthesegeräts 30 mit mittels der Schnittstelle 21 hergestellt.

Die Rekonstruktionseinheit 33 umfasst hier eine integrierte Anpassungseinheit 24. Sei darauf hingewiesen, dass die räumliche bzw. strukturelle Anordnung der Steuerungseinrichtung 20, der Rekonstruktionseinheit 33 und der Anpassungseinheit 24 erfindungsgemäß variabel ist, solange ein Datenaustausch zwischen den einzelnen Komponenten stattfinden kann. Die Anordnung kann also beispielsweise hochgradig integriert, also beispielsweise in einer einzelnen Rechnereinheit realisiert sein. In einer anderen Variante kann die Anordnung auch dezentralisiert, also zum Beispiel die Röntgenquellen 36, 36' und der Detektor 32 in einem Untersuchungsraum, die Steuerungseinrichtung 20 hingegen in einem separaten Kontrollraum angeordnet sein. Die Funktionsweise der einzelnen Komponenten 23, 24, 25, 26 wird später anhand von FIG 2 erläutert.

Die Steuerungseinrichtung 20, die Rekonstruktionseinheit 33 sowie die davon umfasste Anpassungseinheit 24 bilden gemeinsam ein Ausführungsbeispiel einer erfindungsgemäßen Ausgleichsvorrichtung 37.

Das Tomosynthesesystem 40 weist ferner ein Terminal 35 auf, das beispielsweise zur Eingabe von Steuerungsvorgaben durch einen Benutzer ausgebildet ist. Auf dem Terminal 35 kann beispielsweise auch ein mit dem erfindungsgemäßen Verfahren ermitteltes Ergebnisbild EB ausgegeben werden. Das Tomosynthesesystem 40 umfasst weiterhin einer Verbindung 41 zu einem externen Netzwerk, wie beispielsweise einem Kliniknetzwerk oder dem Internet, über die eine Fehlerinformation FI, die beispielsweise von der Detektionseinheit 23 der Steuerungseinheit 20 ausgegeben wird, übermittelt werden kann.

In FIG 2 ist ein Ausführungsbeispiel eines erfindungsgemäßen Verfahrens zur Erzeugung eines Ergebnisbildes EB schematisch als Blockdiagramm dargestellt.

In einem ersten Schritt I wird eine mangelhafte, d.h. nicht voll funktionstüchtige, Röntgenquelle 36' mittels der Detektionseinheit 23 detektiert. Dies kann beispielsweise mittels eines im Rahmen einer Widerstandsmessung festgestellt irregulären Spannungsabfalls erfolgen. Mit der Detektion der mangelhaften Röntgenquelle 36' können zugleich auch deren Position bzw. deren benachbarte funktionstüchtige Röntgenquellen 36 ermittelt werden.

In einem weiteren Schritt II werden Werte für die Röntgenströme RI der benachbarten funktionstüchtigen Röntgenquellen 36 ermittelt, die gegenüber den im regulären Betrieb verwendeten Werten erhöht sind. Diese die Werte für die Röntgenströme RI können für einen Folgenden nicht von dem erfindungsgemäßen Verfahren umfassten Akquisitionsschritt IV als Steuerungsparameterwerte mittels der Röntgenstromregelungseinheit 26 an die Röntgenquellen 36 für deren Betrieb übermittelt werden.

In einem Schritt III werden Werte für die Röhrenspannungen RU der benachbarten funktionstüchtigen Röntgenquellen 36 ermittelt, die gegen über den im regulären Betrieb verwendeten Werten gleich oder erhöht sind. Auch sie können für den Akquisitionsschritt IV als Steuerparameterwerte mittels der Röhrenspannungsregelungseinheit 25 an die Röntgenquellen 36 für deren Betrieb übermittelt werden.

Die Ermittlung der der Röhrenspannungen RU sowie der Röntgenströme RI erfolgt in gegenseitiger Abhängigkeit und bevorzugt in Abhängigkeit von der Anzahl und/oder der Position der mangelhaften Röntgenquellen 36', um eine Röntgenintensität bzw. eine Gesamtdosis zu erzielen, wie sie bei einer regulären Untersuchung üblich wäre. Dabei werden insbesondere die Röntgenströme RI bzw. die Röhrenspannungen RU von Röntgenquellen 36 erhöht, die benachbart zu der mangelhaften Röntgenquellen 36' angeordnet sind. Dadurch kann vorteilhafterweise die in diesem Winkelbereich nicht bzw. nicht ausreichend akquirierte Projektionsaufnahme PA der mangelhaften Röntgenquelle 36' kompensiert werden.

In einem Akquisitionsschritt IV, der nicht vom erfindungsgemäßen Verfahren umfasst ist, erfasst der Detektor 32 zu jeder der nacheinander in beliebiger Reihenfolge aktivierten Röntgenquellen 36 eine Projektionsaufnahme PA des Untersuchungsobjekts O. Die Akquisition solcher Projektionsaufnahmen per ist dem Fachmann ordentlich bekannt und soll daher nicht weiter detailliert werden.

In einem weiteren Schritt V wird die Rekonstruktion zum Ausgleich der mangelhaften Röntgenquelle 36' mittels der Anpassungseinheit 24 angepasst. Das heißt Parameterwerte, wie insbesondere die Gewichtung der einzelnen Projektionsaufnahmen PA, werden so verändert, dass das Ergebnisbild EB möglichst dem einer regulären Bildgebung entspricht. Im Rahmen dieses Schrittes V kann auch ein spezifisches Rekonstruktionsverfahren zur Erzeugung des Ergebnisbildes EB ausgewählt werden und/oder Pre- bzw. Post-Processing-Schritte vorgenommen werden, um im Ergebnis möglichst eine reguläre Bildgebung zu erreichen. All diese Anpassungen können beispielsweise unter dem Begriff "Rekonstruktionsparameterwerte" RP zusammengefasst werden und sind als solche speicherbar und für eine gegebenenfalls später oder andernorts durchgeführte Rekonstruktion abrufbar bzw. übermittelbar.

In einem Schritt VI erfolgt die tatsächliche Rekonstruktion des Ergebnisbildes EB auf Basis der Projektionsaufnahmen PA mittels der Rekonstruktionseinheit 33, wobei die Rekonstruktion mit den Schritt V ermittelten Anpassungen bzw. mit den Rekonstruktionsparameterwerten RP durchgeführt wird. Das Ergebnisbild EB kann dabei beispielsweise in Form eines Volumenbildes bzw. eines Schichtbildstapels und/oder in Form eines synthetischen Mammogramms erzeugt werden. Der grundsätzliche Ablauf einer Rekonstruktion ist bekannt, weswegen auf detaillierte Ausführungen verzichtet wird.

In einem weiteren Schritt VII kann die Steuerungseinrichtung 20 bzw. das Tomosynthesesystem eine Fehlerinformation FI betreffend die mangelhafte bzw. defekte Röntgenquelle 36' mittels einer Schnittstelle bzw. Verbindung 41 an ein Netzwerk ausgeben. Die Fehlerinformation FI kann gegebenenfalls zusätzlich auch eine Information über den Gerätestandort umfassen. Sie kann beispielsweise an Service Personal weitergeleitet werden, das eine Instandsetzung des Geräts entsprechend der Anzahl der defekten Röntgenquellen und des Gerätestandortes veranlassen kann.

Alternativ oder zusätzlich kann auf Basis der Fehlerinformation FI einen Umverteilung von anstehenden Untersuchungen mittels Krankenhausplanungssystems erfolgen. Dabei können Untersuchungen mit höheren Anforderungen an die Bildgebung zum Beispiel auf andere Modalitäten umverteilt werden, wobei die Anforderungen an die Bildgebung beispielsweise auf Basis des Krankheitsbildes und/oder der Patientenakte ermittelt werden können. Dadurch kann eine effizientere Nutzung der Modalitäten sichergestellt werden, bei der die ausreichend hohe Qualität des Bildmaterials für die Befundungen weitestgehend erhalten bleibt.

Mit dem erfindungsgemäßen Verfahren zur Erzeugung eines Ergebnisbildes lassen sich somit die Wirkungen von mangelhaften bzw. ausgefallenen Röntgenquellen im Wesentlichen kompensieren, sodass vorteilhafterweise geringere Ausfallzeiten des Tomosynthesegeräts bzw. -systems erzielt werden können. Dadurch können zum einen Kosten eingespart werden, die bei der Anschaffung von zusätzlichen Modalitäten aus Redundanzgründen anfallen würden, und zum anderen kann ein reibungsloser Ablauf bei der Nutzung des Tomosynthesegeräts gewährleistet werden.

Es wird abschließend noch einmal darauf hingewiesen, dass es sich bei den vorhergehend detailliert beschriebenen Vorrichtungen und Verfahren lediglich um Ausführungsbeispiele handelt, welche vom Fachmann in verschiedenster Weise modifiziert werden können, ohne den Bereich der Erfindung zu verlassen. So wurde zuvor beispielhaft lediglich ein Mammographiesystem beschrieben, die Erfindung kann sich jedoch grundsätzlich auf jedes Röntgensystem mit kombiniertem Röntgen-3D-Ultraschall-Gerät beziehen. Weiterhin schließt die Verwendung der unbestimmten Artikel "ein" bzw. "eine" nicht aus, dass die betreffenden Merkmale auch mehrfach vorhanden sein können. Ebenso schließen die Begriffe "Gerät", "Einrichtung" und "System" nicht aus, dass die betreffende Komponente aus mehreren zusammenwirkenden Teilkomponenten besteht, die gegebenenfalls auch räumlich verteilt sein können.

## Patentansprüche

1. Verfahren zur Erzeugung eines Ergebnisbildes (EB) mit einem Tomosynthesegerät (30), welches eine Mehrzahl von stationären Röntgenquellen (36, 36') aufweist, umfassend zumindest folgende Schritte:
- Detektion (I) zumindest einer mangelhaften Röntgenquelle (36'),
- Anpassung (V) einer Rekonstruktion zum Ausgleich der mangelhaften Röntgenquelle (36') und
- Erzeugung (VI) des Ergebnisbildes (EB) mittels der angepassten Rekonstruktion.

2. Verfahren nach Anspruch 1, wobei Gewichtungen der Röntgenquellen (36) bei der Anpassung (V) der Rekonstruktion (VI) umverteilt werden.

3. Verfahren nach einem der vorstehenden Ansprüche, wobei Röntgenströme der funktionsfähigen Röntgenquellen (36) erhöht werden.

4. Verfahren nach Anspruch 3, wobei die Röntgenströme der Röntgenquellen (36), die benachbart zu der mangelhaften Röntgenquelle (36') angeordnet sind, stärker erhöht werden.

5. Verfahren nach einem der vorstehenden Ansprüche, wobei Röhrenspannungen der funktionsfähigen Röntgenquellen (36) erhöht werden.

6. Verfahren nach einem der Ansprüche 3 bis 5, wobei eine Gesamtdosis gleich gehalten wird.

7. Verfahren nach einem der vorstehenden Ansprüche, wobei eine Fehlerinformation (FI) erzeugt und bevorzugt über ein Netzwerk (41) übermittelt wird.

8. Ausgleichsvorrichtung (37) für ein Tomosynthesegerät (30), das eine Mehrzahl von stationären Röntgenquellen (36, 36') aufweist, umfassend
- eine Steuerungseinrichtung (20) aufweisend eine Detektionseinheit (23), die ausgebildet ist, eine mangelhafte Röntgenquelle (36') zu detektieren,
- eine Anpassungseinheit (24), die ausgebildet ist, eine Rekonstruktion (VI) zum Ausgleich der mangelhaften Röntgenquelle (36') anzupassen, und
- eine Rekonstruktionseinheit (33), die ausgebildet ist, ein Ergebnisbild (EB) mittels der angepassten Rekonstruktion zu erzeugen.

9. Tomosynthesegerät (30) mit einer Mehrzahl von stationären Röntgenquellen (36, 36') und einer Ausgleichsvorrichtung (37) nach Anspruch 8.

10. Tomosynthesesystem (40) mit einem Tomosynthesegerät (30) nach Anspruch 9.

11. Computerprogrammprodukt mit einem Computerprogramm, welches direkt in eine Speichereinrichtung einer Ausgleichsvorrichtung (37) ladbar ist, mit Programmabschnitten, um alle Schritte eines Verfahrens nach einem der Ansprüche 1 bis 7 auszuführen, wenn das Computerprogramm in der Ausgleichsvorrichtung (37) ausgeführt wird.

12. Computerlesbares Medium, auf welchem von einer Rechnereinheit einlesbare und ausführbare Programmabschnitte gespeichert sind, um alle Schritte eines Verfahrens nach einem der Ansprüche 1 bis 7 auszuführen, wenn die Programmabschnitte von der Rechnereinheit ausgeführt werden.

## Claims

1. Method for generating a result image (EB) using a tomosynthesis device (30) that has a plurality of stationary X-ray sources (36, 36'), including at least the following steps:
- detecting (I) at least one faulty X-ray source (36'),
- adapting (V) a reconstruction for the purpose of compensating for the faulty X-ray source (36'), and
- generating (VI) the result image (EB) by means of the adapted reconstruction.

2. Method according to claim 1, wherein, when the adaptation (V) of the reconstruction (VI) is carried out, weightings of the X-ray sources (36) are redistributed.

3. Method according to one of the preceding claims, wherein X-ray streams of the functional X-ray sources (36) are boosted.

4. Method according to claim 3, wherein the X-ray streams of the X-ray sources (36) that are arranged adjacent to the faulty X-ray source (36') are boosted to a greater extent.

5. Method according to one of the preceding claims, wherein tube voltages of the functional X-ray sources (36) are increased.

6. Method according to one of claims 3 to 5, wherein a total dose is kept the same.

7. Method according to one of the preceding claims, wherein an item of fault information (FI) is generated and is preferably transmitted over a network (41).

8. Compensation device (37) for a tomosynthesis device (30) that has a plurality of stationary X-ray sources (36, 36'), including:
- a controller (20) having a detection unit (23) that is embodied for detecting a faulty X-ray source (36'),
- an adaptation unit (24) that is embodied for adapting a reconstruction (VI) for the purpose of compensating for the faulty X-ray source (36'), and
- a reconstruction unit (33) that is embodied for generating a result image (EB) by means of the adapted reconstruction.

9. Tomosynthesis device (30) having a plurality of stationary X-ray sources (36, 36') and a compensation device (37) according to claim 8.

10. Tomosynthesis system (40) having a tomosynthesis device (30) according to claim 9.

11. Computer program product having a computer program that may be loaded directly into a storage device of a compensation device (37), having program portions, in order to carry out all the steps of a method according to one of claims 1 to 7 when the computer program is executed in the compensation device (37).

12. Computer-readable medium on which program portions that may be read from a processor unit and executed are stored, in order to carry out all the steps of a method according to one of claims 1 to 7 when the program portions are executed by the processor unit.

## Revendications

1. Procédé de production d'une image (EB) de résultat par un appareil (30) de tomosynthèse, qui a une pluralité de sources (36, 36') fixes de rayons X, comprenant au moins les stades suivants :
- détection (I) d'au moins une source (36') défectueuse de rayons X,
- adaptation (V) d'une reconstruction pour compenser la source (36') défectueuse de rayons X et
- production (VI) de l'image (EB) de résultat au moyen de la reconstruction adaptée.

2. Procédé suivant la revendication 1, dans lequel on répartit des pondérations des sources (36) de rayons X lors de l'adaptation (V) de la reconstruction (VI).

3. Procédé suivant l'une des revendications précédentes, dans lequel on augmente les courants de rayons X des sources (36) de rayons X aptes à fonctionner.

4. Procédé suivant la revendication 3, dans lequel on augmente davantage les courants de rayons X des sources (36) de rayons X qui sont voisines de la source (36') défectueuse de rayons X.

5. Procédé suivant l'une des revendications précédentes, dans lequel on augmente les tensions de tube des sources (36) de rayons X aptes à fonctionner.

6. Procédé suivant l'une des revendications 3 à 5, dans lequel on maintient égale une dose d'ensemble.

7. Procédé suivant l'une des revendications précédentes, dans lequel on produit une information (FI) de défaut et on la transmet de préférence par un réseau (41).

8. Installation (37) de compensation pour un appareil (30) de tomosynthèse, qui a une pluralité de sources (36, 36') fixes de rayons X, comprenant
- un dispositif (20) de commande ayant une unité (23) de détection constituée pour détecter une source (36') défectueuse de rayons X,
- une unité (24) d'adaptation constituée pour adapter une reconstruction (VI) pour compenser la source (36') défectueuse de rayons X et
- une unité (33) de reconstruction constituée pour produire une image (EB) de résultat au moyen de la reconstruction adaptée.

9. Appareil (30) de tomosynthèse, ayant une pluralité de sources (36, 36') fixes de rayons X et une installation (37) de compensation suivant la revendication 8.

10. Système (40) de tomosynthèse ayant un appareil (30) de tomosynthèse suivant la revendication 9.

11. Produit de programme d'ordinateur ayant un programme d'ordinateur, qui peut être chargé directement dans un dispositif de mémoire d'une installation (37) de compensation, comprenant des parties de programme pour effectuer tous les stades d'un procédé suivant l'une des revendications 1 à 7, lorsque le programme d'ordinateur est réalisé dans l'installation (37) de compensation.

12. Support déchiffrable par ordinateur, sur lequel sont mis en mémoire des parties de programme déchiffrables et réalisables par une unité informatique pour effectuer tous les stades d'un procédé suivant l'une des revendications 1 à 7, lorsque les parties de programme sont réalisées par l'unité informatique.
